# EUROPEAN PATENT SPECIFICATION

(11) **EP 3 756 617 B1**
(45) Date of publication and mention of the grant of the patent: **22.05.2024**
(21) Application number: 19182891.2
(22) Date of filing: 27.06.2019
(51) Int. Cl.: A61C 9/00, A61B 6/14, A61B 5/00, A61B 5/22

(54) **METHOD FOR RECORDING AT LEAST ONE ANATOMICAL FEATURE OF A PATIENT**
VERFAHREN ZUR ERFASSUNG VON MINDESTENS EINEM ANATOMISCHEN MERKMAL EINES PATIENTEN
PROCÉDÉ POUR ENREGISTRER AU MOINS UNE CARACTÉRISTIQUE ANATOMIQUE D'UN PATIENT

(43) Date of publication of application: 30.12.2020
(73) Proprietor: DENTSPLY SIRONA Inc., York, PA 17401 (US); Sirona Dental Systems GmbH, 64625 Bensheim (DE)
(72) Inventor: Maur, Susanne, 64625 Bensheim (DE); Hülsbusch, Markus, 64625 Bensheim (DE)
(74) Representative: Özer, Alpdeniz

(56) References cited:
- CN-A- 102 507 052
- CN-A- 105 496 430
- DE-A1-102015 211 166
- KR-A- 20120 069 846
- KR-A- 20180 033 106
- US-A1- 2015 305 669
- US-A1- 2015 327 958
- S LOGOZZO ET AL: "A Comparative Analysis Of Intraoral 3d Digital Scanners For Restorative Dentistry", THE INTERNET JOURNAL OF MEDICAL TECHNOLOGY, vol. 5, no. 1, 1 January 2008 (2008-01-01) , pages 1-18, XP055438348,

## Description

### TECHNICAL FIELD OF THE INVENTION

The present invention relates to a method for recording at least one anatomical feature of a patient in order to control at least one recording apparatus for recording an image of an oral area of a patient.

### BACKGROUND ART OF THE INVENTION

When making recordings, in particular image recordings, of an oral area of a patient, in particular x-ray recordings, the fundamental problem arises that the recording angle and the recording area must be set such that x-ray exposure for a patient is reduced. The recording area should be selected to be as small as possible for this purpose. On the other hand, however, the recording area should record all oral elements essential for the respective diagnosis, such as teeth, bones, jaw, soft parts and similar.

Furthermore, there is the problem that the image recording angle must be selected such that an overlay of different elements, which can lead to unwanted coverages, are avoided. Therefore, a positioning of a patient relative to the recording appliance, in particular an image recording appliance, has to date been achieved by means of a corresponding patient affixation. Through a bite piece, the position of a patient is approximately defined relative to the image recording appliance or the image source.

A selection of the recording area is made by trained personnel and through the manual selection of a pre-designed image recording program. A precise positioning of the recording area is difficult, however, since the positioning is conducted solely through a top view onto the soft part surface of the patient. This can be supported by a positioning of the recording area onto the soft part surface of the patient, although this method remains imprecise and prone to error.

Despite the relevant training, an imprecise selection of the recording area occurs in particular, so that frequently, areas are selected that are too large. Frequently, too large an area is selected by the operator in order to ensure that the relevant anatomy is shown. While this leads to increased x-ray exposure, it is thus ensured that the desired area is shown and repeat recordings to correct the recording area can be avoided. With this method, an optimal setting of the irradiation angle of the image recording radiation cannot generally be achieved.

Reference is made to EP1560520 and EP1793739. Further reference is made to US2015/0305669A and US2015/0327958A1 each disclosing a prior art bite device.

### DISCLOSURE OF THE INVENTION

The objective of the present invention is a method that overcomes these disadvantages of the prior art and which delivers the above advantages.

The objective of the present invention have been achieved through the method as defined in claim 1.

The bite device used in the method of claim 1, has at least one sensor device, by means of which, at least partially, at least one feature of the anatomical structure of the patient is recordable in the area of the bite device and/or the bite area.

Here, it is particularly preferred that the bite area at least partially comprises at least one deformable first material, wherein by means of the sensor device, at least partially, the contour influenced by the bite piece and/or the surface structure of the bite device and/or the bite area is recordable, preferably without contact and/or optically, in particular being closable on the anatomical structure due to the influenced contour and/or surface structure.

Further, in exemplary embodiments it is recommended that by means of the sensor device, in particular outside the bite area, at least partially, the anatomical structure of the patient is recordable without contact and/or optically, in particular directly.

Here, it is particularly preferred that the recording apparatus comprises at least one radiation source and at least one radiation detector, in particular at least one x-ray source and at least one x-ray detector, preferably the recording apparatus is designed as an intra-oral recording apparatus, in particular an intra-oral x-ray apparatus or extra-oral recording apparatus, in particular an extra-oral x-ray apparatus, and/or the recording apparatus is designed as a volume tomography (DTV) recording apparatus.

In one example, the oral area and/or the oral element comprises at least one oral cavity, at least one tooth, at least one occlusal area and/or tooth, at least one buccal area and/or tooth, at least one incisal area and/or tooth, at least one apical area, at least one cervical area, at least one upper jaw, at least one upper jaw arch, at least one lower jaw, at least one lower jaw arch and/or at least one gum.

It is also preferable that the first material is plastically deformable and/or by means of at least one first sensor facility of the sensor device, the contour, surface structure and/or anatomical structure of the patient, is recordable by means of at least partial sensing, recording, measurement and/or scanning of the anatomy of the patient, the bite device and/or of the bite area.

In some examples, the first material comprises, at least partially, the sensor device, in particular the first sensor facility, and/or is actively connected to said device, in particular is in contact with it.

It is also preferred that the first material comprises at least one foam, at least one gel, at least one self-hardening material, at least one shape memory material, and/or at least one dental impression compound.

One advantageous exemplary embodiment of the bite device can be characterized in that the contour, the surface structure and/or the anatomical structure is recordable at least in areas in the labial, lingual, palatinal, buccal, medial, distal, occlusal, incisal and/or cervical direction, in particular in relation to the oral element, and/or the sensor device, in particular the first sensor facility, is disposed at least partially in the labial, lingual, palatinal, buccal, mesial, distal, occlusal, incisal and/or cervical direction, in particular in relation to the oral element.

Further, a bite device according to an example can be characterized by at least one support structure, wherein the first material and/or the sensor device, in particular the first sensor facility, is actively connected to the support structure, at least partially, in particular is or are connected to the support structure, is or are supported by the support structure and/or is or are affixed to the support structure.

With the examples described above, it is particularly preferred that the first sensor device, in particular the first sensor facility, is disposed at least partially between the first and material and the support structure.

With the two examples described above, it is particularly preferred that the support structure, at least partially, comprises at least one second material, wherein the second material is preferably elastically and/or plastically deformable, and/or the second material, at least partially, has a higher degree of rigidity than the first material, and/or the support structure, in particular the second material, has at least one second sensor facility, which is in particular comprised by the sensor device at least partially, wherein preferably, by means of the second sensor facility, at least one partial deformation of the support structure is recordable, in particular qualitatively and/or quantitatively, preferably at least while the patient is biting down.

Further, for the bite device according to one example, it is recommended that the first material and/or the second material comprises, at least partially, at least one piezoelectric, inductive, capacitive and/or resistive material.

It is also recommended that the first sensor facility and/or the second sensor facility comprises or comprise at least one piezoelectric element, at least one inductive element, at least one capacitive element, at least one resistive element, at least one sensor for recording at least one pressure and/or at least one pressure force, at least one sensor and/or transducer for recording at least one distance, at least one optical, acoustic, electro-optical and/or electro-acoustic sensor and/or transducer, in particular an ultrasound and/or infrared sensor and/or transducer, and/or at least one LED and/or at least one laser.

Further, a bite device according to an example can be characterized by at least one connecting element for the at least indirect connection of the bite device in particular of the bite area, the support structure, the sensor device, the first sensor facility and/or the second sensor facility with a recording apparatus, in particular with at least one support element, of the recording apparatus.

With the above example, it is particularly preferred that the connecting element comprises at least one j oint, preferably at least one ball joint, and/or the connecting element is actively connected with at least one third senor facility, which is in particular partially comprised by the sensor facility, in particular comprises the third sensor facility and/or at least one actuator, preferably for moving the bite device relative to the recording apparatus, preferably in particular to the support element.

Finally, for the example above, it is preferred that by means of the third sensor facility, a relative position between the recording apparatus, in particular between the radiation source and/or the radiation detector of the recording device is recordable on the one hand, and the bite device, in particular the bite area, the support structure, the sensor device, the first sensor device and/or the second sensor device is recordable on the other.

Further, another example provides a bite device that is characterized by at least one fourth sensor facility, which is in particular at least partially comprised by the sensor device, wherein by means of the fourth sensor facility, at least one position and/or orientation of the bite device, which is preferably freely movable and disposable within the space, the bite area, the first material of the sensor device, the first sensor facility, the support structure, the second material, the connecting element and/or the third sensor facility, is or are determinable, in particular relative to the recording apparatus, in particular relative to the radiation source and/or the radiation detector.

Here, it is particularly preferred that the fourth sensor facility comprises at least one optical electromagnetic and/or magnetic tracking module and/or at least one inertial module.

Further, another example delivers a recording apparatus comprising at least one radiation source and at least one radiation detector and at least one bite device, which is preferably at least partially disposable in a beam path from the radiation source to the radiation detector, in particular between the radiation source and the radiation detector.

For the recording apparatus, it is in particular recommended that the recording apparatus is designed as an intra-oral apparatus, and extra-oral recording apparatus and/or a DVT recording apparatus.

The object in question is attained by means of a method according to independent claim 1.

Here, it is particularly preferred that the bite area at least partially comprises at least one first deformable material and/or a contour and/or surface structure of the first material, of the bite device and/or of the bite area influenced by the bite piece is recorded and/or determined by the sensor device.

Further, it is recommended for the method that by means of the sensor device, in particular outside the bite area, at least partially, preferably without contact and/or optically, in particular directly, the anatomical structure of the patient is recorded, preferably by means of a, preferably optical and/or acoustic scattering property, by means of at least one density, by means of a pressure force built up by the anatomical structure and/or by at least one direction of the pressure force.

Here, it is particularly preferred that as a feature of the anatomical structure, at least one alignment, at least one expansion, at least one material property, such as artificial material or natural material, at least one oral element of the patient, such as an oral cavity, at least one tooth, at least one tooth crown, at least one occlusal area and/or tooth, at least one buccal area and/or tooth, at least one incisal area and/or tooth, at least one cervical area, at least one upper jaw, at least one upper jaw arch, at least one lower jaw, at least one lower jaw arch and/or at least one gum is or are recorded and/or determined.

For the method according to one example, it is also recommended that by means of the sensor device, the contour and/or surface structure after biting down is recorded, in particular after removal of the bite device from the oral cavity of the patient, preferably after the bite device has been plastically deformed by being bitten on.

As an alternative to the above example, it can be provided that by means of the sensor device, the contour, the surface structure and/or the anatomical structure is recorded during biting down.

Further, it is recommended according to one example, that on the basis of the anatomical feature, the anatomical structure, the surface structure and/or the determined contour, at least one recording parameter, such as a sharp layer, at least one alignment of the recording apparatus, in particular of the radiation source and/or the radiation detector, one ray dose of the radiation source, one recording area of the recording apparatus, an irradiation angle of the recording apparatus, a trajectory of the recording apparatus, an aperture position of the recording apparatus and/or a radiation spectrum of the recording apparatus is or are determined, wherein in particular, the recording apparatus is controlled at least in part on the basis of the recording parameter.

With the two above examples, it is in particular preferred that the recording of the contour, of the surface structure and/or of the anatomical structure is also conducted at least at times while the recording is being made, in particular the anatomical feature and/or the recording parameter is or are altered, adapted, repositioned and/or re-determined, preferably continuously, while the recording is being made.

It is also recommended according to one example that the recording is interrupted when the determined anatomical feature, the contour, the surface contour, the anatomical structure and/or the recording parameter lies or lie outside a predetermined area.

Finally, a method according to one example can be characterized in that as a recording, a digital volume tomography (DVT) recording is made, in particular on the basis of the determined contour, the determined anatomical structure of the determine anatomical feature and/or of the determined recording parameter, a positioning of the volume, a determination of the size of the volume and/or a trajectory of the recording apparatus is or are conducted.

By means of a bite piece with which the anatomy of the patient is recorded, a precise positioning and alignment of a recording area on a patient is enabled.

The information obtained during biting down by means of the bite device or the method according to one example can be used, particularly for extra-oral, 3D recordings, to adapt the volume position and volume size on an anatomy of a patient to be shown. For in particular extra-oral, 2D recordings, this information can be used to adapt the layer position, the irradiation angle and the size of the irradiated area to the anatomy of the patient. The bite device according to the invention also makes it possible to record the patient movement before and during a recording and to include it in the recording.

In the sense of an example, an extra-oral recording or an extra-oral recording apparatus is understood as meaning that both a radiation source and a radiation detector of the recording apparatus are disposed outside of the body of a patient. In particular, it is here provided that the radiation source, in particular the x-ray source, and the radiation detector, in particular the x-ray detector, are affixed to a shared rotation unit. During the recording, the radiation source and the radiation detector rotate along a trajectory around the patient.

In the sense of an example, an intra-oral recording apparatus or an inter-oral recording are understood as meaning that the radiation detector or the radiation source is disposed at least partially within a body opening and/or a body cavity of the patient, such as the oral cavity. Usually, with such intra-oral recording apparatuses, the radiation detector is held in a fixed position, in particular in relation to the bite device, while the radiation source moves relative to the radiation detector and/or to the patient.

Such recording apparatuses usually have a storage unit for the reconstruction or data processing of the data provided by the radiation detector and/or sensor device. It is also possible to compare the stored data with the actual conditions in real life, and thus to develop an adaptive system, so that with subsequent recordings, a selection of the image parameters can be achieved that is further optimized. In particular, the recording apparatus has a processor, in particular of a PC, for processing the data provided by the sensor device and/or for triggering and regulating the recording apparatus. Alternatively, the recording apparatus is actively connected to such a processor, instead of comprising it.

Through the interaction of the bite device with the sensor device, in particular the first sensor facility, it is possible to record an anatomical structure of the patient. This can on the one hand be achieved by recording or recognizing a deformation of the bite piece and by subsequent conclusions drawn about the anatomy of the patient.

On the other hand, as an alternative or a supplement, the anatomical structure of the patient can be recorded through the use of the sensor device in order to directly determine or measure the anatomical structure, i.e. without the "diversion" of recording a deformation. For this purpose, it is beneficial that in the areas in which the anatomical structure of the patient does not touch the bite device, the anatomical structure is measured without contact, in particular by means of optical and/or acoustic measurement methods. Pressure force and the pressure direction in the area of the contact between the anatomical structure and the bite device can also be recorded, in particular by means of the sensor device. As well as recording the distances between the anatomical structure and areas of the bite device, in particular of the sensor device, suitable sensors can also be used to record parameters such as material thicknesses of both the anatomical structure and of the first material.

This relation from the bite device with the sensor device is in particular advantageous when the recording apparatus is an x-ray source. Thus, an x-ray dose onto the patient can be reduced by the necessary amount, without at the same time having to compensate with regard to the recording quality and the necessary recording area. The bite device according to the invention makes it possible in particular to record a plurality of oral elements or oral areas, or by means of the recording apparatus.

Here, it is advantageous when the first material is plastically deformable. Thus, a patient must only bite down on the bite device once and this can immediately be removed from the mouth of the patient, which increases the patient's comfort. The use of a plastic material also makes it possible for the patient to only bite down once on the bite device, and then a movement of the patient, in particular a change to the bite impression, does not influence the measurement result of the sensor device. Here, the sensor device, in particular the first sensor facility, can sense or scan the contour of the plastically deformable first material, while the patient bites down on the bite device or said bite device is located in the oral area of the patient, i.e. online or simultaneously.

In the sense of an example, a contour is understood as being the form or structure of a three-dimensional surface of the respective element, in particular of the first material or of the anatomical structure. In other words, the contour is in particular a surface relief. Alternatively, in this manner, it is possible for the bite device to be sensed or scanned following removal from the oral area of the patient, and thus for the information required for the control of the recording apparatus to be recorded, i.e. offline or at a different time. Further, it is possible that the first material comprises the sensor device or is a part of this sensor device. As a result, it is possible for the information required to be directly obtained from the formation of the first material. In particular, it is here advantageous when the first material is a foam, a gel or a form memory material. All these materials can be elastically deformable or, if a plastic form is required, a self-hardening material is also suitable.

An elastic deformation of the first material is advantageous in that such a material makes it possible for the bite piece to be recorded and evaluated, in particular continuously, during biting down by the patient. Thus, it is possible that movements of the patient can be recorded and that these can be included in the control of the image recording apparatus. This recording of the movement is also possible in cases when the anatomical structure is recorded as an alternative or a supplement, without contact, by means of the sensor device. Preferably, the first material surrounds different oral elements, such as teeth, from different sides, or the sensor device records the contour of the bite piece from different directions of the oral elements. Here, it is particularly advantageous when the contour can be recorded in the labial, lingual, palatinal, buccal, mesial, distal, occlusal, incisal and cervical direction. It is preferable that the bite device comprises a support structure that has a higher rigidity compared to the first material. The sensor device or the first sensor facility, and/or the formable first material, can then be supported or carried by this support structure. For example, a sandwich structure is advantageous in which the sensor device or the first sensor facility is disposed between the support structure and the deformable first material. However, the support structure itself can also be included in the recording of information via the contour of the oral area or for determining the anatomical feature of the patient. Here, it is particularly preferred that the support structure itself comprises an elastically and/or plastically deformable second material and a plastic and/or elastic deformation of the support structure is recordable by means of a second sensor facility. This makes it possible for the contour of the support structure to be adapted to the anatomy of the patient on the one hand, in order to thus improve the values recorded by the first sensor facility, and on the other, the values of the second sensor facility can provide further information about the anatomy of the patient, such as the progression of the sharp layer.

It is preferred that the bite device or the bite area is connected by means of at least one connecting element, such as a sensitive joint, to the recording apparatus or to a support element of the recording apparatus. This makes it possible for the position of the bite device or of the bite area to be recorded in space, and thus a more precise alignment of the recording apparatus, in particular of a recording source, relative to the patient or to the bite device, is made possible. This sensitivity can in particular by provided by the fact that the connecting element comprises at least one third sensor facility.

As an alternative or supplement, a fourth sensor facility can be provided, by means of which a relative position between the bite piece and the recording apparatus is recorded, for example by means of optical tracking. This fourth sensor facility can be used in order to achieve a higher precision in the determination of the relative position of the bite device to the recording apparatus, in addition to the third sensor facility. It could also be provided that use of the connecting element and the third sensor facility is entirely avoided, and the position and alignment of the bite device in space, in particular relative to the recording apparatus, is essentially determined by means of the fourth sensor facility. With a determination of a position relative to the recording apparatus, in particular, a position in the coordinate system of the recording apparatus is determined. In this coordinate system, preferably, the trajectory of the radiation source or of the radiation detector is also described.

In particular, it is advantageous when the method according to the invention is used in order to create a digital volume tomography recording (DVT) of the patient. In particular, such a recording can be used in multiple ways, in order to obtain improved information about the anatomy of the patient with subsequent treatments or further recordings.

In general, therefore, the method according to the invention provides more precisely resolved measurement data regarding the anatomy of the patient, in particular of a jaw arch and the teeth of the patient. It is possible that a bite plate is present as a support structure, on which a sensor device, in particular comprising first sensor facilities in the form of pressure-sensitive sensors, is disposed. Inductive sensors for the spatially resolved recording of contacts of the oral elements, such as teeth or the jaw arch, are used with the bite device and in particular with the bite area, as well as capacitive, resistive, optical or piezo sensors. According to an example, a foam is used, for example, as a deformable first material, in order to bridge the empty spaces that are otherwise present between the sensor device or the first sensor facility and the recorded anatomy of the patient. In particular, the use of the sensor device or the first sensor facility enables the recording of the different compressions of the first material, and thus a conclusion to be made regarding a size of an area of the first material between the sensor facility and the measured anatomy, and thus to determine the distance of the anatomical element from the bite device and thus the position in space, when this distance is not at least partially directly recorded without contact, as described above.

As already mentioned above, the sensor device or the first sensor facility can be attached to a support structure which is a rigid or flexible base. If this base is flexible, and if it is adapted to the bite or anatomy of the patient, through the deformation of the bite device or support structure, a conclusion can already be reached regarding the anatomy, i.e. the position of the oral elements in space, and at the same time, the position of the first sensor facilities can be recorded.

For example, it is possible to record the deformation by measuring shear forces by means of piezo sensors within the support structure.

A particularly simple realization emerges from the fact that a comparatively low-cost bite device is used that comprises a plastically deformable material. Following the creation of the bite piece or the bite registration, the bite device can be removed and the anatomy can be recorded by sensing the plastically deformed material. While the image is being recorded, a bite piece known from the prior art can then be used, by means of which the patient is aligned along a predetermined causal plane and an unequivocally identifiable anatomy, such as front teeth, are positioned.

In a particularly advantageous manner, the sensor device can be read prior to making a DVT recording, in particular by means of the computing unit, in order to support volume positioning. Here, it is particularly preferred when an operator of the image recording apparatus is shown the prepared measurement data such that they can determine the recording area on this basis, or it is possible for the recording apparatus to automatically adapt the recording geometry and/or further recording parameters to the desired recording area. With this adaptation, although already when determining the anatomical structure, other parameters and data can also be included. At least partially, data from preceding and/or existing recordings, in particular x-ray and/or DVT recordings, panorama recordings and/or optical scans can be included, or used as references.

It is also possible to use the bite device for making a panorama recording. This makes it possible to determine an optimum recording apparatus control for a panorama layer recording by means of the data from the sensor device. Thus, in particular, the likely position of the sharp layer for the most orthoradial irradiation possible for optimizing the irradiation angle and the required recording area is enabled.

As already mentioned above, the bite device makes it possible for the sensor device to be continuously read during recording. Thus, the recording apparatus can be readjusted with a recognized patient movement, or even a termination of the recording can be triggered with stronger patient movements.

In particular, the data provided by the bite device enables a measurement of the jaw geometry, so that the radiation parameter can be adapted to the recording parameters required for the recording. For example, the jaw geometry makes it possible to draw conclusions regarding a skull size of the patient, which can in particular be supported by a measurement of the temple support. Thus, the image recording apparatus can be adapted to the respective characteristic data of the patient; for example, it is also possible for a child mode of the image recording apparatus to be automatically activated.

Furthermore, the bite device makes it possible to determine the relative position of the patient and their alignment relative to an apparatus coordinate system, in particular of the recording apparatus. The data recorded by means of the sensor device can also be used to draw conclusions about further features of the anatomical structure of the patient.

Thus, for example, conclusions can be drawn regarding non-measurable areas of the anatomical structure. For example, a tooth crown can be measured and conclusions can be drawn regarding the further progression of the tooth, e.g. its root. Advantageously, for this purpose, the direction of a force exerted by the patient onto the bite device, the first material and/or the sensor device, is evaluated. On the basis of the path of the pressure force, conclusions can then be drawn regarding the progress of the tooth root.

The method according to one example also makes it possible for a material of the oral element to be determined. For example, conclusions can be made between natural teeth and artificial teeth on the basis of the measurement data recorded by the bite device. Further, it is possible to determine whether a material foreign to the tooth, such as a bridge, is present. In particular, conclusions can be drawn on the basis of such properties of the oral elements that scattering properties of the material, in particular in relation to optical and/or acoustic radiation, are recorded and evaluated.

In particular, the bite device further makes it possible to conduct a punctiform adaptation of the recording parameters. For example, for areas of the anatomical structure in which metals or fillings or crowns, or strongly absorbent materials, are used, a radiation dose can be increased.

The main plane of a cone beam geometry of the recording apparatus can also be optimized to the geometry or anatomy shown. Further the irradiation angle and an angle sensing of the recording apparatus, in particular of the radiation detector and/or the radiation source, can be optimized to the anatomy shown.

It is also made possible for a filter that influences the beam quality, in particular location-dependent filters, to be optimized while the recording is being made, in particular during the movement of the recording apparatus or the radiation detector and/or the radiation source, preferably along the trajectory, for the anatomy to be shown.

It is particularly preferred that several bite devices are kept in reserve in different sizes or model sizes, so that the best possible adaptation of the bite device to the respective patient or in particular their jaw size and jaw form, is enabled. Overall, the advantages arise from this that a simplified workflow is achieved when positioning a patient for a recording of an oral area, since the recording apparatus is adapted to the given patient position. On the basis of the adapted positioning of the recording apparatus, in particular of its recording area, thus achieved, smaller volumes and panorama areas can be sensed, which leads to a reduction in dose for the patient. Overall, the quality of the recording generated by the recording apparatus is also increased, since in particular, only the anatomically relevant structures are shown, as a result of which the diagnosis reliability is increased and the number of repeat recordings can be reduced. Further, the image quality is improved due to the fact that an optimized irradiation angle and an optimized layer position is achieved. In particular, the radiation parameters can be adapted to the anatomy of the patient, since both the further anatomy of the patient can be included in the control of the image recording apparatus and beyond this, an adaptation is enabled when the patient moves during the recording.

### BRIEF DESCRIPTION OF THE DRAWINGS

Further features and advantages of the invention as defined by the appended method claim 1, emerge from the description below, in which exemplary embodiments are explained with reference to schematic drawings,
in which:
Figure 1 shows a top view onto a bite area of a first embodiment of a bite device according to the invention, which comprises a plastically deformable first material;
Figure 2a shows a schematic cross sectional view of a bite area of a second embodiment of a bite device during biting down by a patient;
Figure 2b shows a schematic depiction of the data recorded by means of the sensor device in the bite device shown in Figure 2a;
Figure 3 shows a perspective top view onto a third embodiment of a bite device;
Figure 4 shows a schematic cross sectional view of a fourth embodiment of a bite device during biting down by a patient;
Figure 5a shows a schematic top view onto a fifth embodiment of a bite device with an essentially rigid support structure;
Figure 5b shows a schematic top view onto a sixth embodiment of a bite device with an essentially deformable support structure;
Figure 6 shows a visualization of the measurement data recorded by means of a bite device; and
Figure 7 shows a further visualization of the measurement data recorded by means of a bite device.

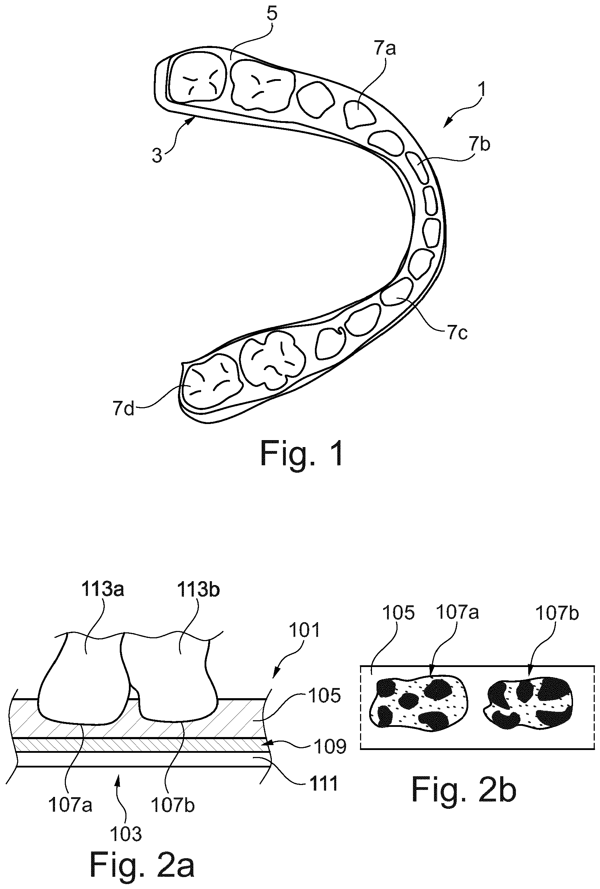

Figure 1 shows a first embodiment of a bite device 1.

The bite device 1 comprises a bite area 3 that comprises a first, plastically deformable material 5. The reference numbers shown in the drawings denote the elements as listed below and will be referred to in the subsequent description of the embodiments.
- 1.: Bite device
- 3: Bite area
- 5: Material
- 7a, 7b, 7c, 7d: Impression
- 101: Bite device
- 103: Bite area
- 105: Material
- 107a, 107b: Impression
- 109: Sensor facility
- 111: Support structure
- 113a, 113b: Tooth
- 201: Bite device
- 203: Bite area
- 205: Material
- 209: Sensor facility
- 211: Support structure
- 215: Connecting element
- 217: Joint
- 219: Support element
- 221: Sensor facility
- 301: Bite device
- 303: Bite area
- 305a, 305b: Material
- 307a, 307b: Impression
- 309a, 309b: Sensor facility
- 311: Support structure
- 313a, 313b: Tooth
- 323a, 323b: Occlusal area
- 325a, 325b: Lingual area
- 327a, 327b: Buccal area
- 401: Bite device
- 403: Bite area
- 405a, 405b: Material
- 407a, 407b: Impression
- 411: Support structure
- 413a, 413b: Tooth
- 423a, 423b: Occlusal area
- 501: Bite device
- 503: Bite area
- 505a, 505b: Material
- 507a, 507b: Impression
- 511: Support structure
- 513a, 513b: Tooth
- 523a, 523b: Occlusal area
- 529: Sensor facility
- 631: Bite piece
- 633: Selection area
- 635: Tooth
- 637: Tooth
- 735: Tooth
- 737: Tooth
- 739: Tooth

In Figure 1, the bite device 1 is shown in a state after a patient has bitten onto the bite area 3 and the bite device 1 has been removed from the mouth of the patient. Due to the plastic deformability of the first material 5, the impressions 7a, 7b, 7c, 7d of oral elements can be clearly in seen in Figure 1 in the form of teeth, which are not shown in Figure 1. Due to the fact that at the point in time at which the patient bites down, the bite device 1, as explained below, was disposed in a predetermined position in space, through sensing of the impressions 7a to 7d, a conclusion can be reached regarding the anatomy of the patient, in particular the position of the teeth in space.

For this purpose, it is provided that the bite device 1 is fed to a sensor device not shown, which analyses, in particular senses, the bite device 1, in particular in the bite area 3. The position of the teeth is then determined from the contour of the bite device 1. On the basis of this data, the patient is then returned to the predetermined position and on the basis of the recorded values, recording parameters are set for making a recording. The recording parameters are in particular a radiation dose and a recording area, in order to be able to depict the oral area of the patient as required.

Figure 2a shows a second embodiment of a bite device 101 according to the invention in a schematic profile view. The reference numerals for the bite device 101 that correspond to those of the bite device 1 bear the same reference numerals but increased by 100.

In contrast to the bite device 1, in the bite device 101, a first material 105 is used which is elastically deformable. The first material 105 is a foam material, for example.

Furthermore, the bite device 101 has a sandwich-type structure. Below the first material 105, a first sensor facility 109 of a sensor device is disposed. This first sensor facility 109 is in turn supported by a support structure 111.

In the state shown in Figure 2a, the patient bites down on the bite device 101 such that oral elements are impressed into the first material 105 in the form of teeth 113a, 113b. From this, the impressions 107a and 107b in the first material 105 result. By means of the sensor facility 109, it is now possible to record the deformation of the first material 105 and thus the contour of the bite device 101 or the bite area 103 in a spatially resolved manner.

In embodiments not shown, the first material is not present, at least partially. By means of the sensor facility 105, the anatomical structure, such as the teeth 113a, 113b, is then recorded or measured without contact, in particular optically.

Figure 2b schematically shows which measurement data is delivered by the first sensor facility 109. On the basis of the measurement data, the scale to which the first material 105 is compressed can be seen. The darker the depiction of the impression 107a or 107b shown in Figure 2b, the stronger the material 105 has been compressed. Conversely, it can therefore be derived how high the chewing surfaces of the teeth 113a and 113b is during biting down.

In the alternative embodiment named above, the position and form of the chewing surface is determined without contact, in particular using optical sensing.

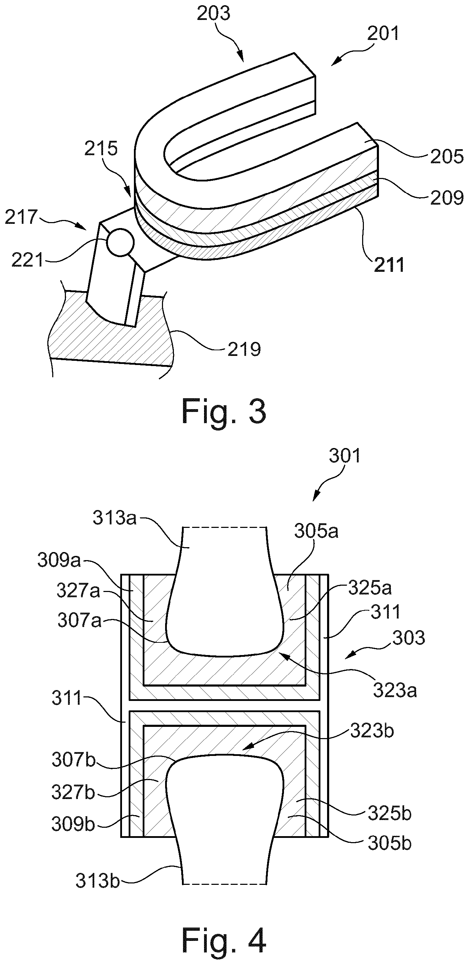

Figure 3 shows a third embodiment of a bite device 201 according to the invention. The elements for the bite device 201 that correspond to those of the bite device 101 bear the same reference numerals but increased by 100.

The bite device 201 is designed such that when a patient bites down, the contour or anatomical feature of the upper jaw of the patient can be recorded. For this purpose, the bite device 201 has a first material 205 in the bite area 203 below which a first sensor facility 209 is disposed. The sensor facility 209 and the first material 205 are supported by a support structure 211.

The support structure 211 is connected via a connecting element 215, which comprises a joint 217 in the form of a ball joint, with a support element 219 of a recording apparatus otherwise not shown.

In order to record the relative position of the bite device 201 in space, in particular relative to the support element 219 and thus relative to the recording apparatus, the joint 217 has a third sensor facility 221. By means of the sensor facility 221, which is advantageously comprised by the sensor device, the position can be determined of the bite device 201, in particular of the bite area 203 in space.

This means that by means of the data provided by the first sensor facility 209 and the second sensor facility 221, a determination of the position of the bite device 201 and thus the anatomical features of the patient when a patient bites down, the position of the oral elements of the patient in space is in particular made possible.

Figure 4 shows a fourth embodiment of a bite device 301 according to the invention. The elements for the bite device 201 that correspond to those of the bite device 201 bear the same reference numerals, but increased by 100.

In contrast to the embodiments of the bite devices 1, 101, 201 described above, the bite device 301 enables the simultaneous recording of the anatomical features of both a lower jaw and an upper jaw of a patient. For this purpose, the bite device 301 has first materials 305a and 305b in the bite area 303.

The materials 305a, 305b can be identical, but also different in their properties, in particular with regard to their rigidity and deformability. When a patient bites down on the bite device 301, an impression 307a is produced in the material 305a by the tooth 313a of an upper jaw of the patient, while an impression 307b is produced in the material 305b by the tooth 313b of a lower jaw of the patient. In order to record the position of the teeth 313a and 313b, the first sensor facilities 309a and 309b not only record an occlusal area 323a and 323b, but also a lingual area 325a and 325b and a buccal area 327b and 327a. In other words, the sensor facility 309a and 309b is disposed both in the occlusal area and in the lingual area and the buccal area of the teeth 313a and 313b. As a result, an even more precise determination of the position and location of the teeth 313a and 313b is enabled, as a result of which an adjustment of the recording apparatus is improved.

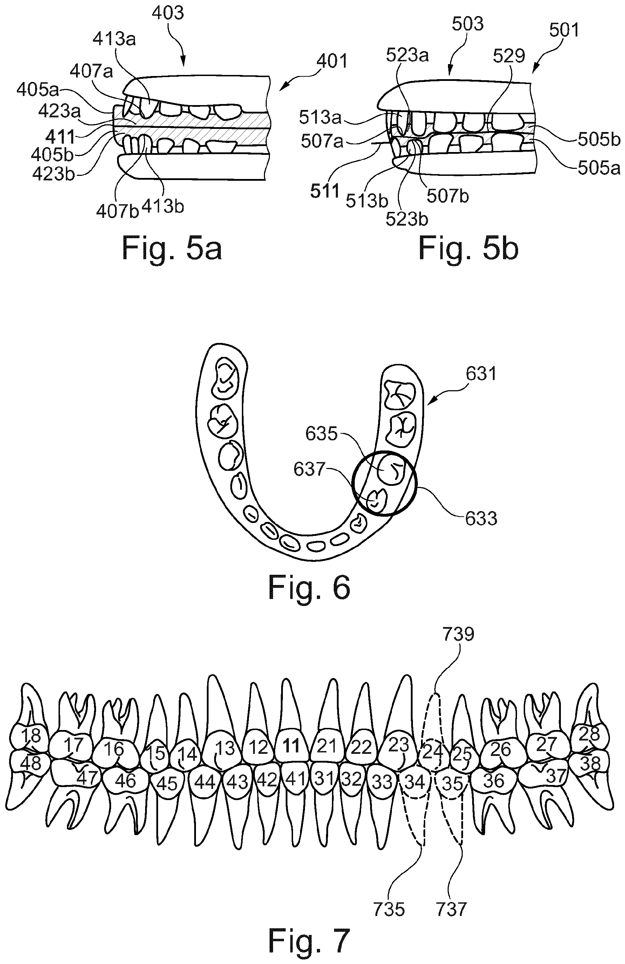

Figure 5a shows a schematic top view onto a fifth embodiment of a bite device 401 according to the invention. The elements for the bite device 401 that correspond to those of the bite device 301 bear the same reference numerals but increased by 100.

For the purpose of simplification of the depiction, the first sensor facility is not shown in Figure 5a. As can be seen from Figure 5a, the support structure 411 is a structure that has a second material, which has a higher rigidity than the first material 405a and 405b, and which is not essentially deformed when bitten on by the patient.

By contrast, Figure 5b shows a sixth embodiment of a bite device 501 according to the invention. The elements for the bite device 501 that correspond to those of the bite device 401 bear the same reference numerals but increased by 100.

In contrast to the bite device 401, the sixth embodiment of the bite device 501 according to the invention comprises a support structure 511 that is formed from a second material, which has a higher rigidity than the first materials 505a or 505b, but which unlike the second material of the support structure 411 is elastically deformable. A second sensor facility 529 is integrated into the support structure 511, by means of which the deformation of the support structure 511 can be recorded.

As a result, a comparatively strong compression or deformation of the materials 505a and 505b is enabled, which means that the measurement precision of the first sensor facility not shown in Figure 5b is increased. Thus, the measurement values are significantly higher than the measurement interference, as a result of which the quality of the measurement values is increased.

Thus, an even more precise determination of the position of the oral elements of the patient, in particular of the teeth 513a and 513b, is possible.

This means that the visualization of the anatomy of the patient, as is shown for example in Figure 6, becomes even better and more precise. Figure 6 shows the bite piece 631 of a patient. A user can now make a selection via a selection area 633 in the area of the bite 631, of which a recording or image depiction is required. On the basis of the data of the bite piece 631, in particular the position, size and alignment of the oral elements 635 and 637, the position of the recording apparatus, its radiation dose and a recording angle is determined such that the best possible recording of the oral elements 635, 637 is enabled, for example in the form of teeth.

Alternatively, the data recorded by means of the bite device 1, 101, 201, 301, 401, 501 according to the invention can also be visualized in the manner shown in Figure 7. Thus, a display of a tooth scheme, i.e. a schematic depiction of the teeth of the patient, similar to a panorama recording, is shown, and a selection is made by selecting the teeth 735, 737. These are thus shown darker than other teeth in the visualization shown in Figure 7. The advantage of the visualization in Figure 7 is that oral elements of the upper jaw, for example of the tooth 739, can also be shown, and also, these elements, in particular the tooth 739, can be selected for a subsequent recording.

The features disclosed, shown and/or described in the above encompass different exemplary embodiments, the scope of invention being solely defined by the appended claim 1.

## Claims

1. A method of determining and recording at least one anatomical feature of a patient, and of controlling an x-ray apparatus for making an x-ray recording of an oral area of the patient, comprising the steps:
- preparing at least one bite device (101, 201, 301, 401, 501),
wherein the bite device is suitable for use with the x-ray apparatus for recording the image of at least one oral area of the patient, and comprises one bite area (103, 203, 303, 403, 503),
which at least partially comes into contact with at least one oral element of the patient when bitten down by the patient; and
at least one sensor device (109, 209, 309, 405, 529), by means of which, at least partially, at least one feature of the anatomical structure of the patient is recordable in the bite area;
- biting down of the patient on the bite area with at least one oral element,
- recording of at least one anatomical structure by means of the sensor device,
wherein the anatomical feature is determined on the basis of the recorded anatomical structure, **characterized in that**
on the basis of the anatomical feature, at least one recording parameter, including a sharp layer, alignment of a radiation source or a radiation detector, one x-ray dose of the radiation source, one recording area of the x-ray apparatus, an irradiation angle of the x-ray apparatus, an aperture position of the x-ray apparatus or a radiation spectrum of the x-ray apparatus is or are determined, wherein the x-ray apparatus is controlled at least in part on the basis of the determined recording parameter, and
(i) the recording of the anatomical structure is also conducted at least at times while the x-ray recording is being made, wherein the anatomical feature is repositioned and a recording parameter is re-determined while the recording is being made, and *if* the anatomical feature is repositioned and a recording parameter is re-determined, *then*
(ii) the recording is interrupted when the repositioned anatomical feature and the re-determined recording parameter lie outside a predetermined range.

2. . The method according to claim 1, **characterized in that** the x-ray recording apparatus is designed as an intra-oral apparatus, and extra-oral recording apparatus or a DVT recording apparatus.

## Patentansprüche

1. Verfahren zum Ermitteln und Aufnehmen von mindestens einem anatomischen Merkmal eines Patienten und zum Steuern einer Röntgeneinrichtung zum Erzeugen einer Röntgenaufnahme eines oralen Bereichs des Patienten, die folgenden Schritte umfassend:
- Herstellen von mindestens einer Aufbissvorrichtung (101, 201, 301, 401, 501), wobei die Aufbissvorrichtung zur Verwendung mit der Röntgeneinrichtung zum Aufnehmen des Bildes von mindestens einem oralen Bereich des Patienten geeignet ist, und
einen Aufbissbereich (103, 203, 303, 403, 503) umfasst, der mindestens teilweise in Kontakt mit mindestens einem oralen Element des Patienten kommt, wenn vom Patienten darauf gebissen wird; und
mindestens eine Sensorvorrichtung (109, 209, 309, 405, 529), mittels derer mindestens ein Merkmal der anatomischen Struktur des Patienten zumindest teilweise in dem Aufbissbereich aufgenommen werden kann;
- Aufbeißen des Patienten auf den Aufbissbereich mit mindestens einem oralen Element,
- Aufnehmen von mindestens einer anatomischen Struktur mittels der Sensorvorrichtung, wobei das anatomische Merkmal auf Basis der aufgenommenen anatomischen Struktur ermittelt wird, **gekennzeichnet dadurch, dass**
auf Basis des anatomischen Merkmals mindestens ein Aufnahmeparameter, einschließlich einer scharfen Schicht, Ausrichtung einer Strahlungsquelle oder eines Strahlungsdetektors, einer Röntgendosis der Strahlungsquelle, eines Aufnahmebereichs der Röntgeneinrichtung, eines Einstrahlungswinkels der Röntgenvorrichtung, einer Blendenposition der Röntgeneinrichtung oder eines Strahlungsspektrums der Röntgeneinrichtung, ermittelt wird oder werden, wobei die Röntgeneinrichtung zumindest teilweise auf Basis des ermittelten Aufnahmeparameters gesteuert wird, und
(i) das Aufnehmen der anatomischen Struktur zumindest zeitweise auch während des Erzeugens der Röntgenaufnahme durchgeführt wird, wobei das anatomische Merkmal neu positioniert und ein Aufnahmeparameter neu ermittelt wird, während die Aufnahme erzeugt wird, und *wenn* das anatomische Merkmal neu positioniert und der Aufnahmeparameter neu ermittelt wird, *dann*
(ii) wird die Aufnahme unterbrochen, wenn das neu positionierte anatomische Merkmal und der neu ermittelte Aufnahmeparameter außerhalb einer vorgegebenen Spanne liegen.

2. . Verfahren nach Anspruch 1, **gekennzeichnet dadurch, dass** die Röntgenaufnahmeeinrichtung als eine intra-orale Einrichtung und extra-orale Aufnahmeeinrichtung oder eine DVT-Aufnahmeeinrichtung gestaltet ist.

## Revendications

1. Procédé de détermination et d'enregistrement d'au moins une caractéristique anatomique d'un patient, et de commande d'un appareil de radiographie pour réaliser un enregistrement radiographique d'une zone buccale du patient, comprenant les étapes de :
- préparation d'au moins un dispositif à mordre (101, 201, 301, 401, 501), dans lequel le dispositif à mordre est adapté pour être utilisé avec l'appareil de radiographie pour enregistrer l'image d'au moins une zone buccale du patient, et comprend
une zone à mordre (103, 203, 303, 403, 503), qui vient au moins partiellement en contact avec au moins un élément buccal du patient lorsqu'elle est mordue par le patient ; et
au moins un dispositif capteur (109, 209, 309, 405, 529), au moyen duquel, au moins partiellement, au moins une caractéristique de la structure anatomique du patient peut être enregistrée dans la zone à mordre ;
- morsure du patient sur la zone à mordre avec au moins un élément buccal,
- enregistrement d'au moins une structure anatomique au moyen du dispositif capteur, dans lequel la caractéristique anatomique est déterminée sur la base de la structure anatomique enregistrée, **caractérisé en ce que**
sur la base de la caractéristique anatomique, au moins un paramètre d'enregistrement, incluant une couche nette, l'alignement d'une source de rayonnement ou d'un détecteur de rayonnement, une dose de rayons X de la source de rayonnement, une zone d'enregistrement de l'appareil de radiographie, un angle d'irradiation de l'appareil de radiographie, une position d'ouverture de l'appareil de radiographie ou un spectre de rayonnement de l'appareil de radiographie est ou sont déterminés, dans lequel l'appareil de radiographie est commandé au moins en partie sur la base du paramètre d'enregistrement déterminé, et
(i) l'enregistrement de la structure anatomique est également réalisé au moins à des moments où l'enregistrement radiographique est en cours, dans lequel la caractéristique anatomique est repositionnée et un paramètre d'enregistrement est re-déterminé alors que l'enregistrement est en cours, et si la caractéristique anatomique est repositionnée et qu'un paramètre d'enregistrement est re-déterminé, alors
(ii) l'enregistrement est interrompu lorsque la caractéristique anatomique repositionnée et le paramètre d'enregistrement re-déterminé se situent en dehors d'une plage prédéterminée.

2. . Procédé selon la revendication 1, **caractérisé en ce que** l'appareil d'enregistrement radiographique est conçu comme un appareil intra-buccal et un appareil d'enregistrement extra-buccal ou un appareil d'enregistrement TVN.
